# EUROPÄISCHE PATENTSCHRIFT

(11) **EP 3 701 913 B1**
(45) Veröffentlichungstag und Bekanntmachung des Hinweises auf die Patenterteilung: **06.04.2022**
(21) Anmeldenummer: 19159566.9
(22) Anmeldetag: 27.02.2019
(51) Int. Cl.: A61F 2/34

(54) **HÜFTPFANNE EINER HÜFTGELENK-ENDOPROTHESE**
ACETABULAR CUP FOR A HIP JOINT ENDOPROSTHESIS
CAVITÉ COTYLOÏDE D'UNE ENDOPROTHÈSE DE L'ARTICULATION DE LA HANCHE

(43) Veröffentlichungstag der Anmeldung: 02.09.2020
(73) Patentinhaber: ARTIQO GmbH, 59348 Lüdinghausen (DE)
(72) Erfinder: Bücken, Ulrich, 59348 Lüdinghausen (DE); Frank, Thomas Mario, 7000 Eisenstadt (AT)
(74) Vertreter: Gosdin, Carstensen & Partner Patentanwälte Partnerschaftgesellschaft mbB

(56) Entgegenhaltungen:
- EP-A1- 0 444 382
- EP-A1- 2 193 764
- WO-A1-2008/015289
- WO-A2-2008/015285
- WO-A2-2008/015286
- DE-C1- 4 220 462

## Beschreibung

Die Erfindung betrifft eine Hüftpfanne einer Hüftgelenk-Endoprothese, umfassend einen äußeren Pfannenabschnitt mit einer konvexen Oberfläche zur Kontaktnahme mit dem Acetabulum und einen inneren Pfannenabschnitt mit einer im Wesentlichen konkaven Oberfläche zur Kontaktnahme mit einem Inlay der Hüftgelenk-Endoprothese, wobei zumindest über einen Oberflächenabschnitt der Hüftpfanne der äußere Pfannenabschnitt relativ zum inneren Pfannenabschnitt in eine radiale Richtung federelastisch gestaltet ist.

Eine gattungsgemäße Hüftpfanne wird in der DE 42 20 462 C1, in der EP 0 444 382 A1, in der EP 2 193 764 A1 und in der WO 2008/015285 A2 offenbart.

Eine weitere Hüftpfanne ist beispielsweise in der EP 0 222 159 A2 beschrieben. Im Englischen wird die Hüftpfanne als "cup" oder "shell" bezeichnet und besteht zumeist aus Metall. Sie wird in das Acetabulum, d. h. in die natürliche Hüftpfanne des Beckenknochens, implantiert, wobei der Aufnahmebereich für die Hüftpfanne kugelabschnittsartig vorgefräst werden kann. Der äußere Pfannenabschnitt mit seiner konvexen Oberfläche ist demgemäß im implantierten Zustand im Kontakt mit dem Beckenknochen, während der innere Pfannenabschnitt mit seiner (im Wesentlichen) konkaven Fläche einen Aufnahmeraum für ein Inlay der Prothese bildet.

Das Inlay wird in die Konkavität der zumeist metallischen Hüftpfanne eingesetzt und mit dieser über eine Inlaykupplung oder Innenkupplung kraftschlüssig verbunden. Es nimmt den metallischen oder keramischen Hüftkopf des femoralen Teils der Hüftprothese auf.

Dabei ist vorgesehen, dass die Prothesen-Hüftpfanne mit Übermaß ("Pressfit") in das Acetabulum implantiert, d. h. in dieses eingeschlagen wird. Daher besitzen üblicherweise zu implantierende Hüftpfannen im Durchmesser ein Übermaß von ca. 2 % bis 3 % gegenüber dem gefrästen Acetabulum. Dies bedeutet, dass beispielweise eine Hüftpfanne mit einer Größe von 50 mm einen effektiven Durchmesser von beispielsweise 51 mm besitzt.

Demgemäß wird durch das Übermaß beim Einschlagen der Hüftpfanne eine gewünschte Vorspannung (d. h. der "Pressfit") erzeugt. Die zumeist metallische Hüftpfanne soll sich beim Einschlagen im knöchernen, ausgefrästen Acetabulum möglichst gleichmäßig verspannen, ohne dass an der Innenkontur ein nennenswerter Verzug, beispielsweise eine ovale Entrundung, eintritt.

Der Pressfit ist gemeinsam mit einer möglichst rauen (konvexen äußeren) Oberfläche der Hüftpfanne für die Primärstabilität der künstliche Hüftpfanne verantwortlich. Das Pressfit ist im Bereich des Pfannenrandes am stärksten und nimmt zum Pfannenboden (d. h. zum Pol) hin ab.

Die Sekundärstabilität der Hüftpfanne wird nach 6 bis 12 Wochen durch das Einwachsen von Knochen in die raue Oberfläche erreicht, gleichzeitig wird die Vorspannung (d. h. der Pressfit) durch Umbauvorgänge des Knochens abgebaut.

Bekannt ist es, dass Pressfitpfannen modular aufgebaut sind. Dies bedeutet, dass in die metallische Hüftpfanne (mittels einer konischen oder sphärischen Kupplung) Inlays aus Kunststoff und/oder Keramik eingebracht werden können. Die konischen Innenkupplungen unterliegen (vor allem für die Aufnahme von keramischen Inlays) hohen Genauigkeiten in Bezug auf Winkeltoleranzen, Durchmesser und Rundheit.

Da die Inlays nach dem Einschlagen in die Pfanne eingesetzt werden (es besteht eine entsprechende Auswahlmöglichkeit verschiedener Inlaytypen je nach intraoperativer Situation), darf der Einschlagvorgang einschließlich des Pressfit die geforderte Genauigkeit der Inlaykupplung nicht beeinträchtigen. Gleichzeit ist beim Einschlagen der Hüftpfanne ein gutes Setzverhalten derselben gefordert. Dies wird (neben der Außengeometrie) von der Wandstärke der Hüftpfannen-Schale beeinflusst.

Dünnwandig Pfannen mit einem elastischen Verhalten der Schale (Federwirkung) erreichen das angestrebte günstigere Setzverhalten gegenüber dickwandigen bzw. steifen Pfannen. Unterschiede in der Knochenhärte können auch besser toleriert werden.

Ein Sortiment von Pressfitpfannen umfasst meist 10 Größen von Durchmesser 46 bis 64. Um das Inlaysortiment so klein wie möglich zu halten, werden oft zwei oder mehrere Hüftpfannen-Kopfgrößen mit einer äußeren Inlaykontur ausgestattet. Dies führt zu variierenden Wandstärken innerhalb eines Pfannensystems, so dass sich ein größenspezifisches Einschlagverhalten ergibt. Des Weiteren haben verschiedene Pfannensteifigkeiten in Zusammenhang mit variierenden Durchmessern einen Einfluss auf die Spannung im umgebenden Knochen und eventuell auf die Umbauvorgänge des Knochengewebes.

Der vorliegenden Erfindung liegt die **Aufgabe** zugrunde, eine Hüftpfanne der eingangs genannten Art so weiterzubilden, dass diese ein einheitliches Setz- bzw. Einschlagverhalten und eine Formstabilität des konkaven inneren Pfannenabschnitts über das gesamte zum Einsatz kommende Größenspektrum aufweist. Demgemäß soll sich eine beim Setzen und/oder Einschlagen erfolgende Formänderung des äußeren Pfannenabschnitts nicht unmittelbar auf die Kontur des inneren Pfannenabschnitts übertragen.

Die **Lösung** dieser Aufgabe durch die Erfindung ist dadurch gekennzeichnet, dass die Federelastizität durch einen Wandungsabschnitt des äußeren Pfannenabschnitts gebildet wird, der zu einem Wandungsabschnitt des inneren Pfannenabschnitts unter Bildung eines Hohlraums mit radialem Abstand verläuft, wobei der federelastisch gestaltete Oberflächenabschnitt um den gesamten Umfang der Hüftpfanne verläuft und frei von federelastischen Elementen ist, wobei sich der federelastisch gestaltete Oberflächenabschnitt auf eine Höhe beschränkt, die maximal 60 %, vorzugsweise maximal 50 %, der gesamten Höhe der Hüftpfanne beträgt, und wobei die Hüftpfanne samt den Wandungsabschnitten einstückig ausgebildet ist.

Die Hüftpfanne besteht bevorzugt aus Metall.

Dabei ist insbesondere vorgesehen, dass sie durch einen additiven Fertigungsprozess hergestellt ist. Hierunter ist insbesondere, aber nicht ausschließlich, der 3-D-Druck zu verstehen, mit dem es in besonders vorteilhafter Weise möglich ist, die gesamte Struktur der Hüftpfanne einteilig zu fertigen.

Somit ergibt sich vorteilhaft eine Außenschale der Hüftpfanne, die eine besondere nachgiebige Außenkontur aufweist, während die Innenschale der Hüftpfanne eine hohe Formstabilität und somit eine stabile Innenkontur aufweist.

Mit der vorgeschlagenen Lösung werden verschiedene Vorteile erreicht:
Die Hüftpfanne hat im Bereich der Pressfit-Wirkung eine vorteilhafte elastische Charakteristik (d. h. Federwirkung) an der Außenschale, so dass ein ideales Setzverhalten und eine einheitliche Spannung im Knochenlager über das gesamte Größenspektrum erreicht werden.

Die Innenkontur der Hüftpfanne ist für die Aufnahme von modularen Inlays geeignet. Somit sind die Anforderungen für Keramik- oder PE-Inlays erfüllt.

Die durch das Pressfit entstehenden Spannungen wirken nicht auf die Innenkontur; insbesondere werden keine Verformungen der Innenkontur hervorgerufen.

Weiterhin werden hohe Spannungsspitzen vermieden, welche in situ beispielsweise beim Laufen oder Stolpern auftreten können, so dass derartige Spannungsspitzen nicht vollständig auf die Inlay-Kupplung wirken.

Vorteilhaft wird, wie erläutert, ein additives Fertigungsverfahren (insbesondere der 3-D-Druck) eingesetzt, so dass eine einstückige Gestaltung der Hüftpfanne möglich ist. Hierdurch können die Vorteile einer elastischen Außenschale der Hüftpfanne mit den Anforderungen einer modularen Inlay-Kupplung kombiniert werden.

In der Zeichnung sind Ausführungsbeispiele der Erfindung dargestellt. Es zeigen:
- Fig. 1: in einer Radialschnittdarstellung eine in das Acetabulum, d. h. in die Beckenpfanne, eingeschlagene Hüftpfanne, die Bestandteil einer Hüftgelenk-Endoprothese ist, wobei die Hüftpfanne gemäß einer nicht erfindungsgemäßen Ausführungsform ausgebildet ist,
- Fig. 2: in einer Radialschnittdarstellung analog zu Figur 1 das Acetabulum mit an dieses angesetzter, aber noch nicht eingeschlagener Hüftpfanne, wobei die Hüftpfanne nach einer Ausführungsform gemäß der Erfindung ausgebildet ist,
- Fig. 3: in einer Radialschnittdarstellung die Hüftpfanne gemäß einer weiteren Ausführungsform der Erfindung,
- Fig. 4: in einer Radialschnittdarstellung einen Teil der Hüftpfanne, wobei eine (nicht erfindungsgemäße) wabenartige Federstruktur zwischen zwei Wandungsabschnitten angeordnet ist,
- Fig. 5: in einer Radialschnittdarstellung einen Teil der Hüftpfanne, wobei eine (nicht erfindungsgemäße) wellenförmig ausgebildete Federstruktur zwischen zwei Wandungsabschnitten angeordnet ist, und
- Fig. 6: in einer Radialschnittdarstellung einen Teil der Hüftpfanne, wobei eine (nicht erfindungsgemäße) säulenartig ausgebildete Federstruktur zwischen zwei Wandungsabschnitten angeordnet ist.

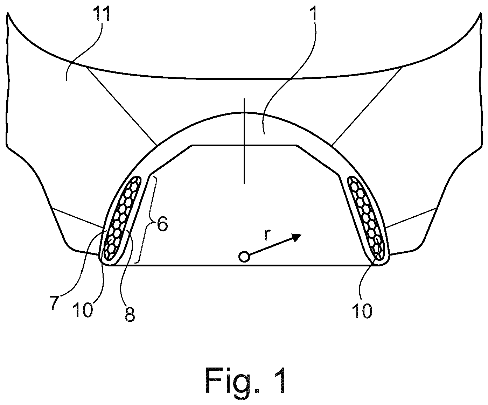

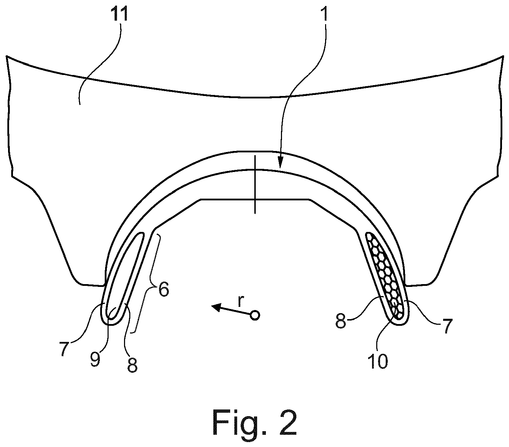

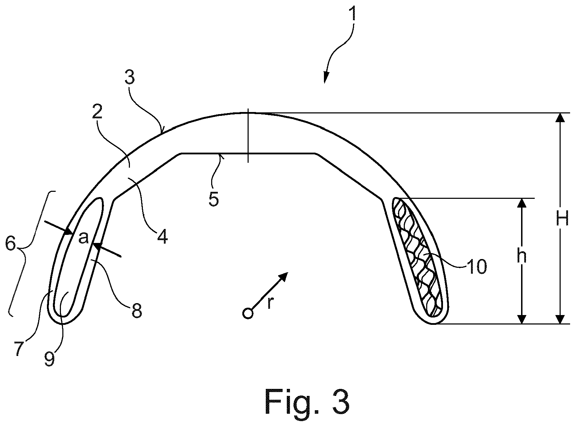

In Figur 1 ist ein Acetabulum 11 schematisch zu erkennen, in welches ein Teil einer Hüftgelenk-Endoprothese, nämlich eine metallische Hüftpfanne 1 implantiert wurde. In der Zusammenschau mit den Figuren 2 und 3 ergibt sich, dass die Hüftpfanne 1 einen äußeren Pfannenabschnitt 2 aufweist, der eine konvexe Oberfläche 3 hat. Diese kontaktiert im implantierten Zustand das Acetabulum 11. Weiterhin hat die Hüftpfanne 1 einen inneren Pfannenabschnitt 4, der im Wesentlichen eine konkave Oberfläche 5 hat und damit einen in etwa halbkugelförmigen Raum umschließt, in dem ein (nicht dargestelltes) Inlay der Hüftgelenk-Endoprothese angeordnet wird (zum Inlay finden sich unten noch nähere Erläuterungen).

In den Figuren 1 bis 3 dargestellt ist ein Radialschnitt durch die Hüftpfanne 1, d. h. ein Schnitt durch die Hüftpfanne 1 entlang eines Großkreises der halbkugelartigen Struktur der Hüftpfanne 1. Demgemäß ergibt sich eine radiale Richtung r von einem gedachten Mittelpunkt der halbkugelartigen Struktur der Hüftpfanne 1.

In der Zusammenschau der beiden Figuren 1 und 2 lässt sich auch der erzeugte "Pressfit" ersehen, der vorliegt, wenn die Hüftpfanne 1 in das Acetabulum 11 eingeschlagen ist. In Figur 2 ist die Hüftpfanne zunächst noch nicht eingeschlagen und lediglich an das Acetabulum 11 angesetzt, woraus ersichtlich ist, dass hier ein radiales Übermaß der Hüftpfanne 1 relativ zum Aufnahmeraum im Acetabulum 11 vorliegt. In Figur 1 ist der eingeschlagene Zustand zu sehen, gemäß dem nun die Hüftpfanne 1 mit einer gewünschten Vorspannung im Acetabulum 11 angeordnet ist.

Am oberen Pol der Hüftpfanne 1 ist nach dem Einschlagen zumeist noch etwas Spiel vorhanden, da postoperativ mit Aufnahme der vollen Belastung eine gewisse Setzung der Hüftpfanne 1 zu erwarten ist.

Beim Einschlagvorgang ist nun angestrebt, dass sich lediglich die laterale Außenschale (Wandungsabschnitt 7 des äußeren Pfannenabschnitt 2, s. unten) verformt, während die mediale Innenschale (Wandungsabschnitt 8 des inneren Pfannenabschnitt 4, s. unten) sich möglichst wenig verformt.

Um dies zu erreichen, ist vorgesehen, dass über einen Oberflächenabschnitt 6 der Hüftpfanne 1 der äußere Pfannenabschnitt 2 relativ zum inneren Pfannenabschnitt 4 in radiale Richtung r federelastisch gestaltet ist. Hierzu wird zunächst auf Figur 3 Bezug genommen, aus der hervorgeht, dass sich der Oberflächenabschnitt 6 über eine Höhe h erstreckt und in diesem Bereich doppelwandig ausgebildet ist, d. h. er weist einen Wandungsabschnitt 7 des äußeren Pfannenabschnitt 2 und einen Wandungsabschnitt 8 des inneren Pfannenabschnitts 4 auf. Zwischen den beiden Wandungsabschnitten 7 und 8 ist ein Hohlraum 9 ausgebildet, der in Umfangsrichtung über die gesamte Hüftpfanne 1 umläuft. Angegeben ist ein radialer Abstand a über den die beiden Wandungsabschnitte 7 und 8 voneinander beanstandet sind.

Zu sehen ist auch, dass die Höhe h des insoweit federelastisch gestalteten Oberflächenabschnitts 6 nur einen Teil der gesamten Höhe H der Hüftpfanne 1 ausmacht; die diesbezüglich bevorzugte Obergrenze sind 60 % der Höhe H.

Die Hüftpfanne 1 ist durch ein additives Fertigungsverfahren, insbesondere durch 3-D-Druck, hergestellt, so dass der Hohlraum 9 im Inneren der einstückigen Struktur der Hüftpfanne 1 leicht ausgebildet werden kann.

In den Figuren 1 bis 3 sind dabei verschiedene Möglichkeiten vorgesehen, wie in Bezug auf die Einstellung der Federelastizität zwischen den beiden Wandungsabschnitten 7 und 8 vorgegangen werden kann.

In der linken Bildhälfte von Figur 2 wie auch in der linken Bildhälfte von Figur 3 ist der Hohlraum 9 vollständig frei, d. h. leer. Damit ergibt sich insoweit eine hohe Elastizität zwischen dem äußeren Wandungsabschnitt 7 und dem inneren Wandungsabschnitt 8.

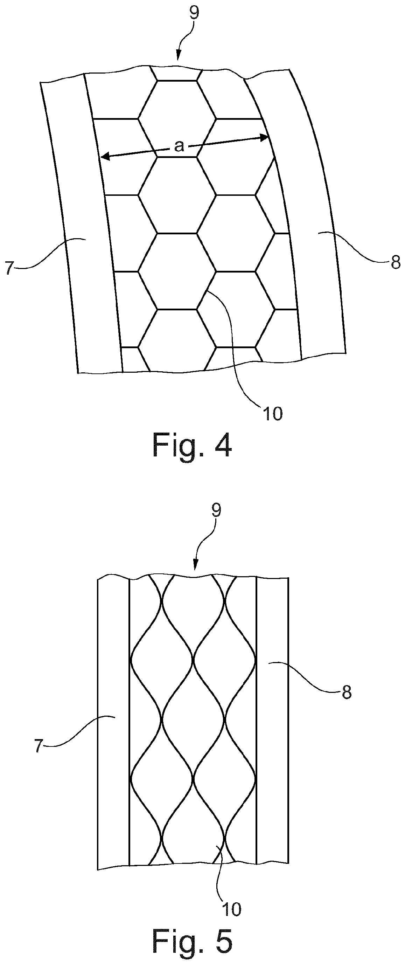

In Figur 1 und auch in der rechten Bildhälfte von Fig. 2 ist indes zu sehen, dass im Hohlraum 9 ein federelastisches Element 10 angeordnet ist, welches eine Wabenstruktur hat, wie sie in Figur 4 nochmals im Detail wiedergegeben ist. Durch das federelastische Element 10 kann die Federelastizität zwischen den beiden Wandungsabschnitten 7, 8 beeinflusst werden, wobei abhängig von der gewählten Struktur des federelastischen Elements 10 die Federrate (Federkonstante) eingestellt werden kann.

Insoweit wird klar, dass es der genannte Hohlraum 9 nicht zwingend macht, dass er gänzlich frei von Material ist; er wird im Rahmen der vorliegenden Beschreibung auch dann als Hohlraum bezeichnet, wenn sich in ihm das nicht erfindungsgemäße federelastische Element 10 befindet. Das federelastische Element 10 ist vorzugsweise einstückig mit dem beidseitig angeordneten Schalenmaterial der Hüftpfanne 1 ausgebildet, was in einfacher Weise per additiver Fertigung erfolgen kann.

In der rechten Bildhälfte von Figur 3 ist eine alternative Ausgestaltung des federelastischen Elements 10 zu sehen, wobei hier eine wellenförmige Struktur vorgesehen ist, die in Figur 5 vergrößert dargestellt ist. Wiederum gilt, dass durch die Gestaltung des federelastischen Elements 10 die Federrate eingestellt werden kann.

Hinsichtlich der Figuren 2 und 3 sei angemerkt, dass hier lediglich zur Illustration eine unterschiedliche Gestaltung des Hohlraums 9 skizziert wurde. Im Regelfall wird der zirkulär umlaufende Hohlraum 9 stets gleichförmig ausgebildet sein, d. h. entweder hohl oder mit einer einheitlichen Struktur des federelastischen Elements 10 versehen sein.

Denkbar ist allerdings grundsätzlich auch, zur Erzeugung eines anisotropen Elastizitätsverhaltens über unterschiedliche Umfangsabschnitte der Hüftpfanne 1 eine verschiedene Gestaltung des Hohlraums 9 vorzusehen.

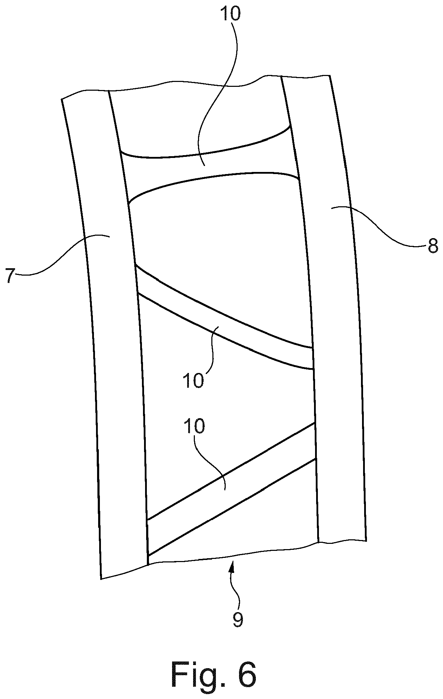

In Figur 6 ist eine andere Möglichkeit der Ausgestaltung der nicht erfindungsgemäßen federelastischen Elemente 10 dargestellt. Hier ist eine Anzahl säulenartiger Streben zu erkennen, die sich entweder in radiale Richtung r (oberste Strebe in Figur 6) oder unter einem Winkel hierzu (mittlere und untere Strebe in Figur 6) erstrecken und so die Federkonstante (d. h. Federrate) zwischen den beiden Wandungsabschnitt 7 und 8 beeinflussen. Der Winkel zur radialen Richtung r kann zwischen 20° und 60° liegen.

Die Gestaltung des Hohlraums 9 sowie der in der Regel vorgesehenen federelastischen Elemente 10 wird fachmännisch so vorgenommen, dass eine gewünschte Federrate zwischen den Wandungsabschnitt 7 und 8 vorliegt. Generell ist dabei angestrebt, die Außenschale (d. h. den Wandungsabschnitt 7 des äußeren Pfannenabschnitts 2) mit einer gewünschten Nachgiebigkeit zu versehen, während die Innenschale (d. h. den Wandungsabschnitt 8 des inneren Pfannenabschnitts 4) möglichst formstabil zu halten, womit die oben erläuterten Vorteile erreicht werden können.

Die zum Einsatz kommenden Inlays sind zumeist modular gestaffelt und werden in verschiedenen Größenabstufungen vorgehalten. Hierbei sind unterschiedliche Hüftpfannen-Größen relevant, die das Inlay aufnehmen. Weiterhin können verschiedene Außendurchmesser bzw. Außenkonturen vorgesehen sein, die exakt die Innendurchmesser bzw. Innenkontur der aufnehmenden metallischen Hüftpfanne passen müssen. Bei den Inlays werden üblicherweise mehrere Hüftkopf-Aufnahmedurchmesser mit einer identischen Außenkontur ausgestattet, so dass sie in ein und dieselbe metallische Hüftpfanne passen. Daraus ergeben sich dann natürlich entsprechende unterschiedliche Wandstärken des Inlays.

Betreffend die Hüftpfanne und das Inlay sind verschiedene Werkstoffkombinationen möglich. Das Inlay kann aus PE-Kunststoff oder Keramik bestehen. Folgende Materialpaarungen sind möglich bzw. gebräuchlich: Metall auf Polyäthylen (englisch abgekürzt "MoP"), Metall auf Keramik "MoC"), Keramik auf Polyäthylen "CoP" und Keramik auf Keramik ("CoC").

### Bezugszeichenliste:

- 1: Hüftpfanne
- 2: äußerer Pfannenabschnitt
- 3: konvexe Oberfläche
- 4: innerer Pfannenabschnitt
- 5: konkave Oberfläche
- 6: Oberflächenabschnitt
- 7: Wandungsabschnitt des äußeren Pfannenabschnitts
- 8: Wandungsabschnitt des inneren Pfannenabschnitts
- 9: Hohlraum
- 10: federelastisches Element
- 11: Acetabulum

- r: radiale Richtung
- a: radialer Abstand
- h: Höhe des federelastisch gestalteten Oberflächenabschnitts
- H: gesamte Höhe der Hüftpfanne

## Patentansprüche

1. Hüftpfanne (1) einer Hüftgelenk-Endoprothese, umfassend einen äußeren Pfannenabschnitt (2) mit einer konvexen Oberfläche (3) zur Kontaktnahme mit dem Acetabulum und einen inneren Pfannenabschnitt (4) mit einer konkaven Oberfläche (5) zur Kontaktnahme mit einem Inlay der Hüftgelenk-Endoprothese, wobei zumindest über einen Oberflächenabschnitt (6) der Hüftpfanne (1) der äußere Pfannenabschnitt (2) relativ zum inneren Pfannenabschnitt (4) in eine radiale Richtung (r) federelastisch gestaltet ist,
**dadurch gekennzeichnet, dass**
die Federelastizität durch einen Wandungsabschnitt (7) des äußeren Pfannenabschnitts (2) gebildet wird, der zu einem Wandungsabschnitt (8) des inneren Pfannenabschnitts (4) unter Bildung eines Hohlraums (9) mit radialem Abstand (a) verläuft,
wobei der federelastisch gestaltete Oberflächenabschnitt (6) um den gesamten Umfang der Hüftpfanne verläuft und frei von federelastischen Elementen ist,
wobei sich der federelastisch gestaltete Oberflächenabschnitt (6) auf eine Höhe (h) beschränkt, die maximal 60 %, vorzugsweise maximal 50 %, der gesamten Höhe (H) der Hüftpfanne (1) beträgt, und
wobei die Hüftpfanne (1) samt den Wandungsabschnitten (7, 8) einstückig ausgebildet ist.

2. Hüftpfanne nach Anspruch 1, **dadurch gekennzeichnet, dass** sie aus Metall besteht.

3. Hüftpfanne nach Anspruch 1 oder 2, **dadurch gekennzeichnet, dass** sie durch einen additiven Fertigungsprozess hergestellt ist.

## Claims

1. Acetabular cup (1) of a hip joint endoprosthesis, comprising an outer cup section (2) with a convex surface (3) for contacting the acetabulum and an inner cup section (4) with a concave surface (5) for contacting an inlay of the hip joint endoprosthesis, wherein at least along a surface portion (6) of the acetabular cup (1) the outer cup section (2) is made resilient relative to the inner cup section (4) in a radial direction (r),
**characterized in that**
the spring elasticity is formed by a wall portion (7) of the outer cup section (2) extending to a wall portion (8) of the inner cup section (4) forming a cavity (9) with radial clearance (a),
wherein the resiliently configured wall portion (6) extends around the entire circumference of the acetabular cup and is free of resilient elements,
wherein the resiliently configured wall portion (6) is limited to a height (h) which is at most 60 %, preferably at most 50 %, of the total height (H) of the acetabular cup (1), and
wherein the acetabular cup (1) together with the wall portions (7, 8) is formed in one piece.

2. Acetabular cup according to claim 1, **characterized in that** it is made of metal.

3. Acetabular cup according to claim 1 or 2, **characterized in that** it is manufactured by an additive manufacturing process.

## Revendications

1. Cavité cotyloïde (1) d'une endoprothèse de l'articulation de la hanche, ladite cavité cotyloïde comprenant une portion de cavité externe (2) pourvue d'une surface convexe (3) destinée à venir en contact avec l'acétabulum et une portion de cavité interne (4) pourvue d'une surface concave (5) destinée à venir en contact avec un insert de l'endoprothèse de l'articulation de la hanche, la portion de cavité externe (2) étant conçue pour être élastique dans une direction radiale (r) par rapport à la portion de cavité interne (4) au moins sur une portion de surface (6) de la cavité cotyloïde (1),
**caractérisée en ce que**
l'élasticité est formée par une portion de paroi (7) de la portion de cavité externe (2) qui s'étend à une distance radiale (a) d'une portion de paroi (8) de la portion de cavité interne (4) pour former une cavité (9),
la portion de surface élastique (6) s'étendant sur toute la circonférence de la cavité cotyloïde et étant exempte d'éléments élastiques,
la portion de surface élastique (6) étant limitée à une hauteur (h) qui représente 60 % maximum, de préférence 50 % maximum, de la hauteur totale (H) de la cavité cotyloïde (1), et
la cavité cotyloïde (1) étant conçue d'une seule pièce avec les portions de paroi (7, 8).

2. Cavité cotyloïde selon la revendication 1, **caractérisée en ce qu'**elle est en métal.

3. Cavité cotyloïde selon la revendication 1 ou 2, **caractérisée en ce qu'**elle est réalisée par un processus de fabrication additive.
